# EUROPEAN PATENT SPECIFICATION

(11) **EP 1 932 481 B1**
(45) Date of publication and mention of the grant of the patent: **30.06.2010**
(21) Application number: 07254806.8
(22) Date of filing: 12.12.2007
(51) Int. Cl.: A61B 10/02

(54) **Biopsy system with vacuum control module**
Biopsiesystem mit Vakuumkontrollmodul
Système de biopsie avec module de commande sous vide

(30) Priority: 13.12.2006 US 869736 P; 07.12.2007 US 952393
(43) Date of publication of application: 18.06.2008
(73) Proprietor: Ethicon Endo-Surgery, Inc., Cincinnati, OH 45242 (US)
(72) Inventor: Ritchie, Paul G., Loveland, Ohio 45140 (US); Speeg, Trevor W.V., Williamsburg, Ohio 45176 (US); Haberstich, Wells D., Loveland, Ohio 45140 (US); Hibner, John A., Mason, Ohio 45040 (US)
(74) Representative: Tunstall, Christopher Stephen

(56) References cited:
- EP-A- 0 995 400
- US-A- 6 162 187
- US-A1- 2003 199 787
- US-B1- 6 428 487

## Description

### BACKGROUND

The present invention relates to a vacuum system according to the preamble of claim 1. Such a system is known from US-A-6 162 187.

When a suspicious tissue mass is discovered in a patient's breast or another area through examination, ultrasound, MRI, X-ray imaging or the like, it may be necessary to perform a biopsy procedure to remove one or more samples of that tissue in order to determine whether the mass contains cancerous cells. A biopsy may be performed using an open or percutaneous method. Medical devices for obtaining tissue samples for subsequent sampling and/or testing are known in the biopsy art. For instance, a biopsy instrument now marketed under the tradename MAMMOTOME is commercially available from Ethicon Endo-Surgery, Inc. for use in obtaining breast biopsy samples. This device generally retrieves multiple core biopsy samples from one insertion into breast tissue with vacuum assistance.

The following patent documents disclose various biopsy devices: US 6,273,862 issued Aug. 14, 2001;US 6,23 1,522 issued May 15,2001 ;US 6,228,055 issued May 8,2001 ;US 6,120,462 issued September 19, 2000; US 6,086,544 issued July 1 1, 2000; US 6,077,230 issued June 20, 2000; US 6,017,316 issued Jan. 25, 2000; US 6,007,497 issued Dec. 28, 1999; US 5,980,469 issued Nov. 9, 1999; US 5,964,716 issued Oct. 12, 1999; US 5,928,164 issued July 27, 1999; US 5,775,333 issued July 7, 1998; US 5,769,086 issued June 23, 1998; US 5,649,547 issued July 22, 1997; US 5,526,822 issued June 18, 1996; and US Patent Application 2003/0199753 published Oct. 23, 2003 to Hibner et al.

EP 0 995 400 A1 discloses a handheld biopsy device for the collection of soft tissue samples. US 6,162,187 discloses a fluid collection apparatus for controlling fluid communication between a vacuum source and first and second vacuum lines of a surgical device. US 6,428,487 discloses a surgical biopsy system comprising an elongated, hollow piercer and a cutter rotatably and axially positionable relative to the piercer.

Some vacuum-assisted biopsy devices may employ a re-usable control module that includes a vacuum pump and other control apparatus. Such vacuum control modules may be relatively large and heavy, and may be mounted on wheels or on a wheeled platform so that they can be moved from room to room in a surgical area.

While a variety of biopsy systems have been made and used, it is believed that no one prior to the inventors has made or used a biopsy system as described in the appended claims.

### SUMMARY OF THE INVENTION

In a first aspect of the invention, there is provided a biopsy system as recited in claims 3 to 11.

Preferably, the vacuum control module is configured to be filled and carried by a single hand.

Preferably, the conduit is unitary with the canister, such that the canister is packaged with the conduit unitarily therewith.

In a second aspect of the invention, there is provided a vacuum system adapted to be used with a biopsy device as recited in claims 1 and 2.

### BRIEF DESCRIPTION OF THE DRAWINGS

While the specification concludes with claims particularly pointing out and distinctly claiming the present invention, it is believed the same will be better understood by reference to the following description, taken in conjunction with the accompanying drawings in which:

Figure 1 is a schematic illustration of a biopsy system;

Figure 1A is a schematic illustration of a distal portion of a tissue piercing cannula having a vacuum lumen and a cutter lumen, with the distal portion of the cutter shown in the cutter lumen.

Figure 1B is a schematic illustration of the vacuum level provided in a vacuum lumen as a cutter is advanced and retracted in a cutter lumen relative to a tissue receiving aperture.

Figure 2 is a schematic exploded view illustration of certain components of the vacuum control module depicted in Figure 1.

Figure 3 is a schematic illustration of a vacuum canister that can be used with the biopsy system of Figure 1.

Figure 4 is a schematic illustration of the interface of a multilumen cable and a vacuum canister cover.

Figure 5 is a schematic illustration of a control module including a handle and a hinged lid.

Figure 5A illustrates a USB port positioned on the back surface of the control module of Figure 5.

Figure 6 illustrates various steps that can be performed with respect to a USB device inserted into a USB port on a control module.

Figure 7 illustrates a pneumatic circuit that may be used with a biopsy system.

Figure 8 illustrates multiple control states that can be employed in controlling a biopsy device in a biopsy system.

### DETAILED DESCRIPTION

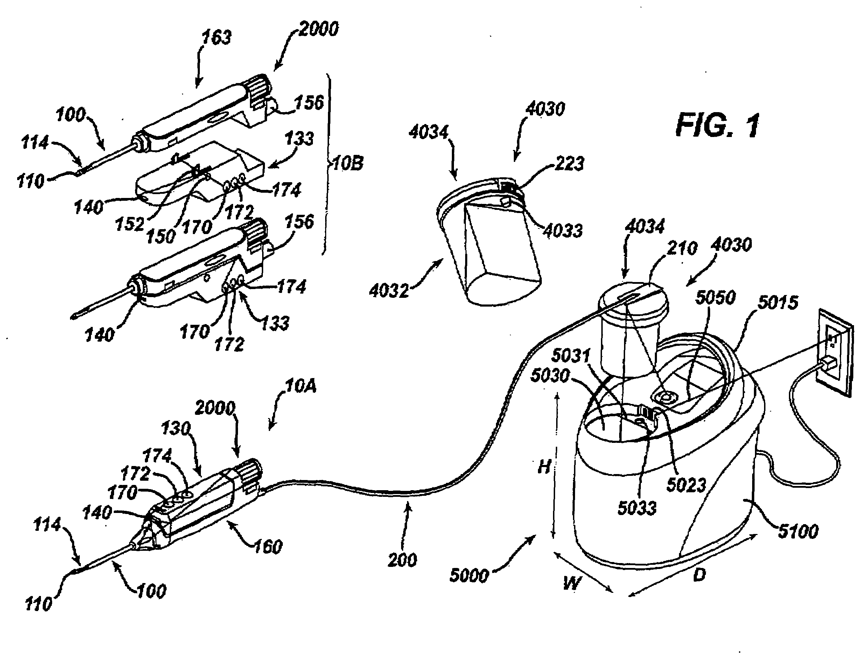

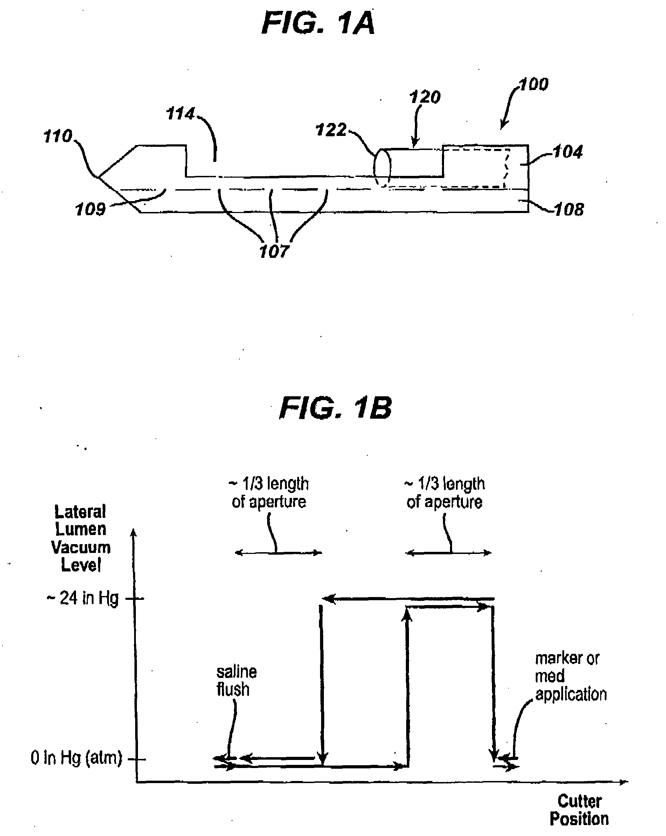

Figure 1 illustrates a biopsy system according to one embodiment of the present invention. The biopsy system of the present example includes a biopsy device 10 having a translating cutter 120 for severing tissue samples, a vacuum control module 5000, and an umbilicus 200 extending from the biopsy device 10 to the control module 5000. The umbilicus 200 can be in the form of a cable having multiple lumens for providing one or more of electrical power, vacuum, pneumatics, hydraulics, or saline to the biopsy device 10. The biopsy device 10 can be a hand held device 10A such as is suitable for use with ultrasound imaging, or alternatively, a stereotactic device 10B configured to be mounted on a stereotactic or X-ray table. The vacuum control module 5000 can be a relatively lightweight, portable unit having a smoothly shaped outer cover 5 100 and a carrying handle 5015. The lightweight control module 5000 of this example can be lifted and moved easily by a single person, with one hand. The multilumen umbilicus 200 of this example provides a single connection between the biopsy device 10 and the control module 5000, eliminating the complexity of having multiple electrical, saline, pneumatic, hydraulic, and/or vacuum lines extending from the biopsy device 10.

As noted above and as shown in Figure 1, biopsy device 10 can be a handheld biopsy device 10A suitable for use with ultrasound imaging. The biopsy device 10A can include a reusable holster 130 and a disposable probe unit 160 that is detachable from holster 130. Together, the holster 130 and the probe 160 form a handpiece that can be comfortably held in and operated with a single hand. Biopsy device 10B can include a disposable probe unit 163, and a reusable stereotactic holster 133 having a firing mechanism for firing a tissue piercing portion of the biopsy device into tissue. The firing mechanism may be power driven (e.g., motorized), and may include a button 150 that may be actuated to activate the firing mechanism; as well as firing members 152 that are configured to engage probe 160 to feed at least a portion of probe 160 into tissue. Any suitable configuration for the firing mechanism may be used, to the extent that a firing mechanism is included at all. The stereotactic holster 133 can be configured for operable mounting onto a stereotactic X-ray table. Of course, biopsy device 1 0A and biopsy device 1 0B may alternatively be used in a variety of other settings or configurations.

The probe units 1 60 and 1 63 of the present example include a distally extending tissue piercing portion, such as a cannula 100 extending distally from 160. The cannula 100 can include a distal tissue piercing tip 1 10 and a tissue receiving aperture 1 14 spaced proximally of the tip 110. The cannula 100 can also include a cutter lumen 104 and a vacuum lumen 108, with passageways 107 and 109 providing flow communication between the lumen 104 and lumen 108 in the distal portion of cannula 100 (Figure 1A). The cannula 100 can be inserted into or adjacent to a tissue mass to be sampled, and the biopsy device 10 is operable to obtain a plurality of severed tissue samples with a single insertion of cannula 100 into tissue.

With cannula 100 being inserted into tissue, tissue drawn into the aperture 114 can then be severed by sharpened distal end 122 (Figure 1A) of a tubular cutter 120 translating within the cannula 100. Vacuum can be applied axially through the cutter 120 and also in vacuum lumen 108 to assist in drawing tissue into aperture 114. Figure 1B illustrates graphically a variable vacuum level that can be provided in vacuum lumen 108 as the cutter 120 is translated relative to the aperture 114. The probe units 160 and 163 can also include a tissue storage assembly 2000, which can be disposed at a proximal end of the probe unit 160, 163 or elsewhere. The tissue storage assembly 2000 can be employed to store multiple tissue samples severed by the cutter translating with the cannula 100 and transferred proximally through the hollow cutter 120 to the tissue storage assembly 2000.

The probe units 160 and 163 of the present example each also include a light 140 positioned near cannula 100. By way of example only, light 140 may comprise an LED or other source of light, and may be configured to at least partially illuminate a site into which cannula 100 is to be inserted. Probe 163 also has a remote thumbwheel 156, which may be rotated to rotate the cannula 100 of probe 163 relative to the remainder of probe 163. Suitable mechanisms for causing such rotation will be apparent to those of ordinary skill in the art in view of the teachings herein. Of course, either probe 160, 163, as well as holsters 130, 133, may be subject to numerous variations and modifications as desired.

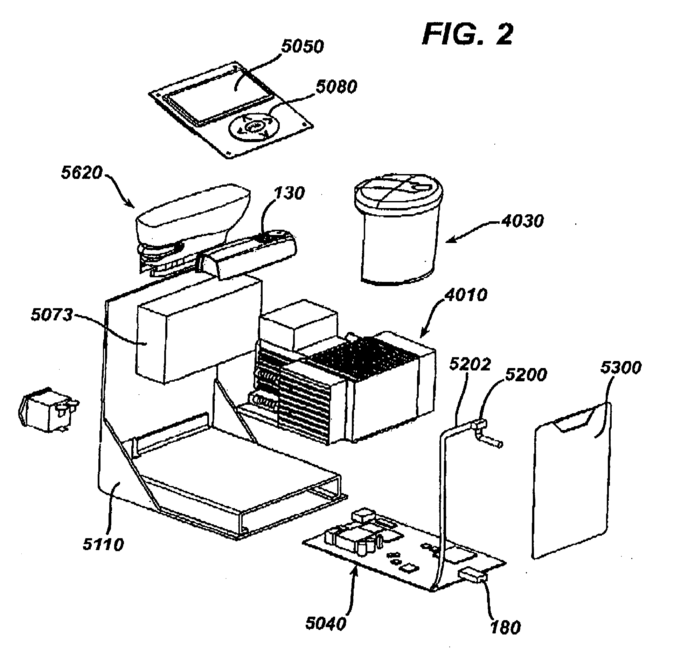

Vacuum control module 5000 of the present example is portable by a single hand, and has a weight of no more than about 18kg (40 pounds), and in one embodiment a weight of less than about 11kg (25 pounds). Alternatively, vacuum control module 5000 is of any other suitable weight. The control module 5000 handle 5015 is shown extending upward from an upper portion of the unit, with the handle 5015 being the upper most component of the control module 5000, as shown in Figure 1. The vacuum control module 5000 of the present example has maximum outer dimensions of width W, depth D, and height H, each of which is less than about 46cm (1.5 feet). Alternatively, vacuum control module 5000 has any other suitable dimensions. Vacuum control module 5000 of the present examples further includes a standard power cord for receiving electrical power from a standard electrical outlet. Figure 2 illustrates various components of the control module 5000 of the present example. The control module 5000 includes an internal aluminum (or other suitable metal) chassis 51 10, which directly or indirectly supports a vacuum pump 4010, an ACDC power supply 5073, and a microprocessor control board 5040. A suitable power supply 5073 includes a 250-Watt power supply model GPFC250 CommerciaVGPFM250 Medical 250 manufactured by Condor D.C. Power Supplies of Oxnard, California. Alternatively, any other suitable power supply 5073 may be used. A suitable vacuum pump 401 0 includes a 2-headed diaphragm pump having a maximum flow rate of less than about 1 8 liters per minute, and providing a maximum vacuum of about 85.0 kPa (25.1 inches of Mercury (Inch Hg.)). Alternatively, vacuum pump 40 10 may have any other suitable components and properties. By way of example only, one suitable vacuum pump 4010 is a model 7006ZVDP-2,3E Diaphragm pump available from Reitschle Thomas, Thomas Products Division of Sheboygan, Wisconsin.

The control module 5000 can also include an LCD display 5050, or other type of display, supported on an outside surface of the control module 5000, or elsewhere (e.g., external to control module 5000, etc.). By way of example only, display 5050 may include a backlit, color LCD display. Alternatively, any other type of display 5050 may be used, or no display 5050 at all. A keypad 5080 may also be provided, near display 5050, on control module 5000. Keypad 5080 may comprise capacitive switches or other input devices, and may be used to enter commands to or otherwise interact with control module 5000. Display 5050 may display operating conditions, menus, or other information, such that display 5050 and keypad 5080 collectively provide a user interface. Of course, a user interface may alternatively be provided using any other suitable components in any other suitable fashion.

In the present example, control module also has an attachment assembly 5200 that is configured to receive an off-the-shelf saline bag 5300. Attachment assembly 5200 is coupled with a flex circuit 5202, and is configured to sense the weight of a saline bag 5300. In particular, control module 5000 may be configured such that it will prevent operation of biopsy device 10 when no weight or insufficient weight is sensed by attachment assembly 5200, which may indicate that no saline bag 5300 is present or that the saline bag 5300 contains an insufficient amount of saline. In addition or in the alternative, control module 5000 may be configured to inform the user, such as via display 5050, that the a saline bag 5300 is not coupled with attachment assembly 5200 or that the saline bag 5300 contains an insufficient amount of saline. Alternatively, data from attachment assembly 5200 may be used in any other suitable way, or attachment assembly 5200 may be omitted altogether.

The vacuum control module 5000 of the present example has a vacuum canister 4030 that is releasably received with an opening 5030 disposed in a generally upward facing outer surface of the control unit 5000. The vacuum canister 4030 may serve as a vacuum "capacitor" for the biopsy vacuum circuit, and can have a volume of less than about 300 cubic centimeters. More particularly, the vacuum canister 4030 can have a volume of about 200 to about 250 cc. Alternatively, vacuum canister 4030 may have any other suitable capacity or properties.

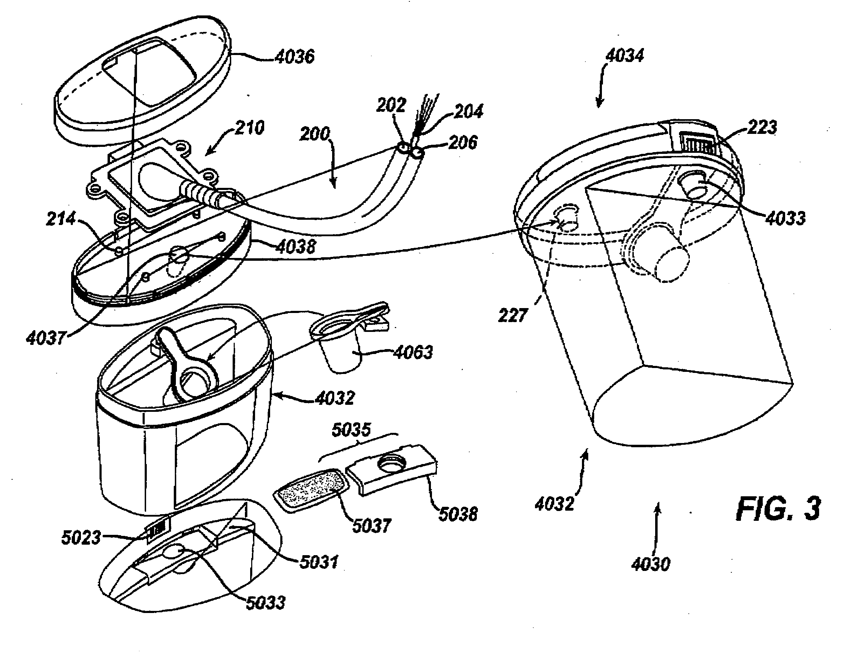

Referring to Figure 3, the umbilicus 200 of the present example can comprise multiple lumens, and can comprise a first lumen 202 for conveying vacuum from the vacuum pump 4010 to the biopsy device 10, a second lumen 204 for conveying one or more electrical conductors for conveying power and control signals from the control module 5000 to the biopsy device 10, and a third lumen 206 for conveying saline. Alternatively, umbilicus 200 may have any other suitable number or type of lumens. As yet another variation, one or more lumens 202, 204, 206 are provided in separate conduits or cables, instead of being integrated into a single umbilicus 200. The proximal end of the umbilicus 200 can include a connection terminator 210. The distal end of the umbilicus 200 can be received in the disposable portion of the biopsy device 10, such as the probe 160 or the probe 163, so that electrical power and control signals are directed through the disposable probe 160, and then to holster 130. One or more controls (e.g., control buttons 170, 172, 174, etc.) can be located on the reusable holster 130. Alternatively, the distal end of the umbilicus 200, or a portion thereof, may be received in holster 130 or elsewhere. Similarly, controls may be located on probe 160, 163 in addition to or in lieu of controls on holster 130.

The vacuum canister 4030 of the present example can include a cup or container shaped body portion 4032 and a lid 4034. The vacuum canister 4030 is configured to be inserted into an opening 5030 in an upwardly facing outer surface of the control module 5000. The canister 4030 can be supported on a lip 503 1 that extends at least partially around the opening 5030. Of course, there are a variety of alternative ways in which vacuum canister 4030 may be configured; as well as alternative ways in which vacuum canister 4030 may engage with control module 5000.

In the present example, the body portion 4032 of the canister 4030 includes a male vacuum port 4033 communicating with the interior of the canister 4030. The port 4033 can sealingly engage a female vacuum port 5033 disposed in an opening in the lip 503 1, when the canister 4030 is inserted into the opening 5030. In other variations, the lip 503 1 or other portion of the control module 5000 may include a male vacuum port 4033; with the canister 4030 having a complimentary female vacuum port 5033. In the present example, the vacuum port 5033 can be connected with a flexible hose or tube or other conduit that communicates with the outlet of the vacuum pump 4010 disposed within the control module 5000. Accordingly, when the canister 4030 is inserted into the opening 5030 in the present example, a vacuum connection is established from the vacuum pump 401 0 to the interior of the canister 4030. The lid 4034 of the present example is adapted to receive the connection terminator 210 disposed at the proximal end of the umbilicus 200. The lid 4034 can include a upper, first lid portion 4036 and a lower, second lid portion 4038. The connection terminator 210 can be captured between the first and second lid portions 4036,4038, and the two lid portions 4036,4038 joined together (such as by a snap fit, by adhesive, or by any other suitable means). The position of the terminator 210 between the lid portions 4036, 4038 can be established by guide pins 2 12 on the lower lid portion which mate with corresponding guide holes 21 4 disposed around the perimeter of the terminator 210. Alternatively, any other suitable structures or features may be used to establish the position of the terminator 210 between the lid portions 4036, 4038, If any are used at all. Indeed, lid 4034 may instead be formed of a single piece instead of two lid portions 4036, 4038, and umbilicus 200 may be secured relative thereto in any other suitable fashion. The multilumen umbilicus 200 and the vacuum canister 4030 can be provided as separate disposable items or provided together as a unitary disposable item.

As shown in Figures 1, 3, and 4, the connection terminator 210 of the present example provides a multi-contact electrical connection 223, which faces outward from the lid 4034 when the terminator 210 is positioned on the lid 4034. The control module 5000 includes a mating multi-contact electrical connection 5023. The multi-contact connection 5023 is disposed adjacent the opening 5030 of the control module 5000, such that when the vacuum canister 4030 is positioned in the opening 5030, the electric contact is established between the control module 5000 and the multilumen umbilicus 200 via the contacts 223 and 5023. It will be appreciated, however, that electrical contact may be provided between control module 5000 and umbilicus 200 using a variety of alternative structures and techniques.

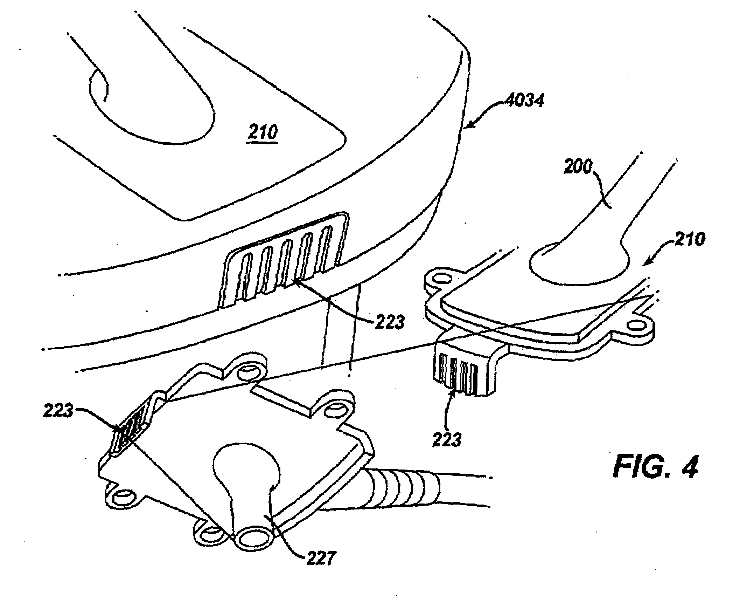

Referring to Figures 3 and 4, the connection terminator 210 can further include a downward facing male vacuum port 227 extending from a bottom surface of the connection terminator 210. The vacuum port 227 communicates with the vacuum lumen 202 in umbilicus 200. When terminator 210 is disposed on lid 4034 between lid portions 4036 and 4038, the vacuum port 227 extends through an opening 4037 in the lower lid portion 4038 to communicate with the interior of the vacuum canister 4030. Alternatively, vacuum lumen 202 may communicate with the interior of the vacuum canister 4030 using any other suitable structures or techniques.

Accordingly, in the present example, the vacuum canister 4030, terminator 210, and control module 5000 are configured such that positioning the canister 4030 in the opening 5030 of the control module 5000 provides an electrical connection and vacuum communication between the biopsy device 10 and the vacuum control module 5000. In other words, fluid and electrical connections between biopsy device 10 and the vacuum control module 5000 are established merely by inserting the canister 4030 into opening 5030, such that additional tube connections or cable connections (e.g., connection of a tube with the canister 4030), etc. need not be established by the user before or after canister 4030 is inserted into opening 5030. As used herein, the term "fluid" should be read to include a vacuum, pressurized air, atmospheric air, liquids (e.g., saline, blood, etc.), and the like, regardless of whether solid materials (e.g., tissue samples or particles, etc.) are conveyed therewith.

Still referring to Figures 3 and 4, a float 4063 can be positioned in the canister 4030. The float 4063 is operable to close vacuum port 4033 in the event fluid accumulates in the canister above an acceptable level. A filter assembly 5035 including a filter pad 5037 and filter pad cover 5038 can be provided at the vacuum port 5033 If desired. Of course, like other components described herein, float 4063, filter assembly 5035, filter pad 5037, and filter pad cover 5038 are all merely optional, and may be modified, substituted, supplemented, or omitted as desired.

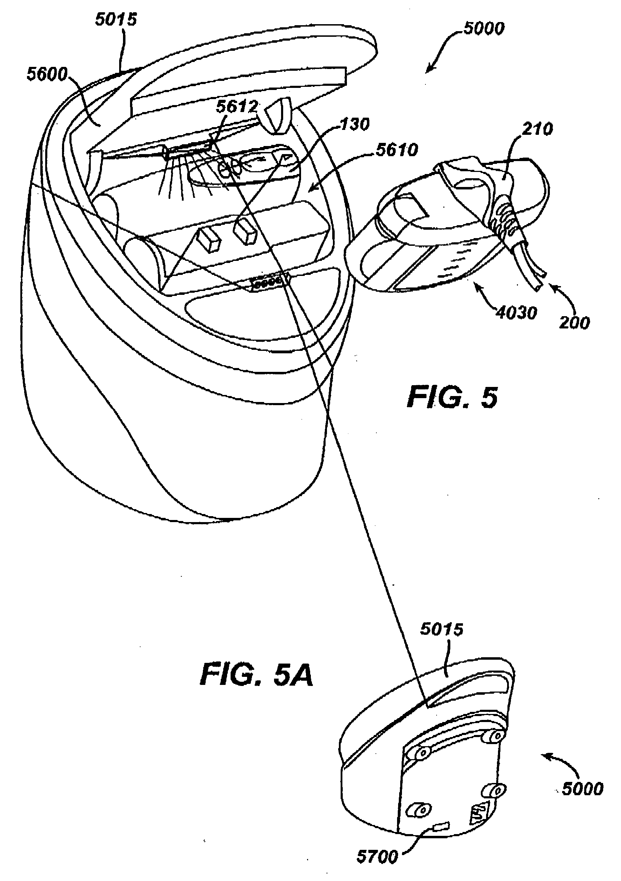

Figure 5 provides a schematic illustration of an embodiment of the control module 5000 that includes a hinged cover 5600. Cover 5600 covers a storage cavity 5610, similar to tissue storage compartment 5620 of the control module shown in Figure 2. The storage cavity 5610 can be sized to store one or more biopsy device holsters 130. If desired, a plurality of UV light sources 5612 (only one shown) can be positioned near or with cavity 5610. Light sources 5612 can be configured to be "on" when the cover 5600 is closed (and "off when the cover 5600 is opened), and can be employed to disinfect or sterilize the item(s) stored in the cavity 561 0. In other embodiments, other components, features, or devices are included to disinfect or sterilize one or more items stored in the cavity 5610, in addition to or in lieu of having light sources 5612. In still other embodiments, no features are included for disinfecting or sterilizing items. Similarly, some variations of control module 5000 lack a cavity 5610 altogether.

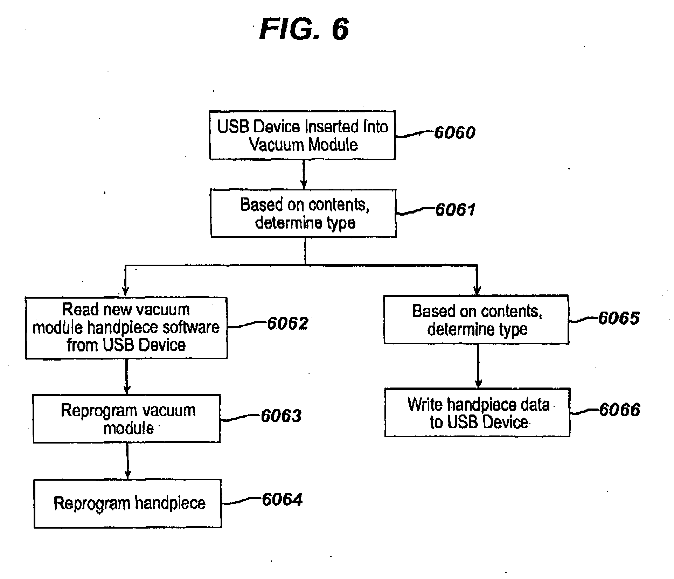

Referring to Figure 5A, one or more Universal Serial Bus (USB) ports 5700 can be provided on an outer surface of the control module 5000. Alternatively, any other type of port (e.g., an SD card slot, an ethernet port, a serial connection port, a proprietary connection port, etc.) may be provided on control module 5000. Figure 6 illustrates a flow chart describing a sequence of operational steps that can be employed when a USB memory device (not shown) is coupled with control module 5000 via port 5700. Based on the contents of the USB memory device, a course of action can be determined. For instance, based on the contents of the USB memory device, the control module microprocessor control may write vacuum control module data to the USB memory device (e.g., usage time, operational status, first use or first in service date, and the like.) The control microprocessor control may also write biopsy device data to the USB memory device (e.g., usage time, operational status, first use, or first in service date). Alternatively, the vacuum control module 5000 may read new calibration dormation, software, or software updates, etc., from the USB memory device, reprogram operation of the vacuum module, and/or reprogram operation of the biopsy device 10. Alternatively, a computer (e.g., desktop or laptop PC), or network (e.g., internet) connection may be made with control module 5000 via port 5700 or in some other fashion.

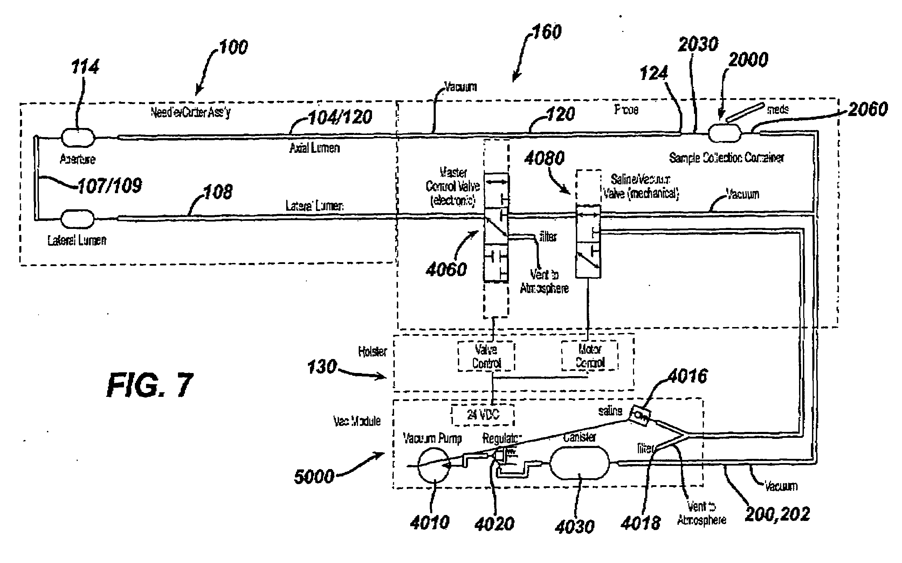

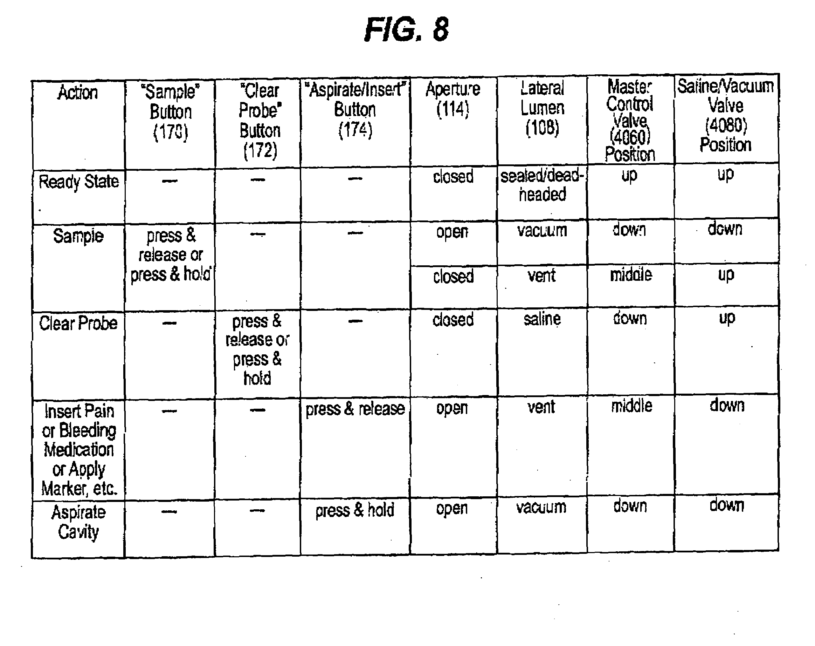

Figure 7 is a schematic illustration of a pneumatic configuration that can be used with the biopsy device 10 and vacuum control module 5000 of the present example. Figure 8 illustrates multiple control states that can be employed in controlling the biopsy device 10.

As shown in Figure 7, the control module 5000 of the present example comprises the vacuum pump 4010, a regulator 4020, and a the vacuum canister 4030 disposed in a pneumatic circuit. A master control valve 4060 and a saline/vacuum valve 4080 are shown schematically as being positioned in the disposable probe 160.

The vacuum provided by vacuum pump 4010 can be directed through the vacuum lumen 202 of umbilicus 200 to the disposable probe 160, to provide vacuum to the cutter 120 and cutter lumen 104. This vacuum can be provided without valving, so that the vacuum provided to the interior of cutter 120 and to cutter lumen 104 of cannula 100 is always "on" when vacuum pump 4010 is operating. Alternatively, one or more valves or similar components may be provided in probe 160, canister 4030, control module 5000, and/or elsewhere. The vacuum provided by umbilicus 200 can also provide a vacuum to the vacuum lumen 108 via the two valves 4060 and 4080. Valve 4080 can be a 3- port12-position valve, with two input ports. One input port can be connected to the vacuum source and the other input port can be connect to a source of saline 4016 (or alternatively vented to atmospheric pressure through filter 401 8). The output port of valve 4080 communicates with an import port of master valve 4060. Other suitable configurations and couplings for the ports will be apparent to those of ordinary skill in the art in view of the teachings herein.

The operational position of the valve 4080 can be configured to correspond to the position of the cutter 120, such as by having cutter 120 extend through the valve 4080 so that the position of the cutter 120 within the valve body determines the operational status (opened or closed) of the valve ports. When the cutter 120 is retracted proximally (e.g., such that tissue aperture 114 is open), the valve 4080 communicates vacuum to the master control valve 4060. When the cutter 120 is advanced distally (e.g., such that tissue receiving aperture 1 14 is closed), the valve 4080 communicates saline to the master control valve 4060. Alternatively, if saline is not available, or not desired, then valve 4080 communicates atmospheric air via filter 401 8 to the master control valve 4060. The valve 4060 can be actuated by a microprocessor controlled motor, by a mechanical link to the cutter 120, or otherwise. The valve 4080 can be spring loaded in one position, and movement of the cutter 120 (such as movement of the cutter 120 to the distal position) can be employed to change the valve operational position. Alternatively, the operational position of the valve 4080 can be configured to correspond to the position of the cutter 120 in any other suitable way. Furthermore, valve 4080 may be configured such that its operational position does not necessarily correspond with the position of the cutter 120.

The master control valve 4060 can be a 3-port/3-position valve. One input port can be connected to the output port of the valve 4080. The second input port can be vented to filtered atmospheric air. The output port of the valve 4060 can be connected to the proximal end of vacuum lumen 1 08 of cannula 1 00. The valve position of valve 4060 can be controlled by the operator of the biopsy device 10 using one or more user control interfaces, such as the control buttons 170, 172, 174 listed in Figure 8 or any other interface. The control buttons 170, 172, 174 can be located at any convenient position on the body of the biopsy device 10, including for instance on handpiece 130, or elsewhere. The valve 4060 can be actuated by a solenoid, motor, via a link to the cutter 120, or otherwise.

With reference to Figure 8, the "Ready State" of biopsy device 10 corresponds to the cutter 120 being advanced to its distal most position and tissue aperture 114 being closed. In the Ready State, the valve 4080 communicates saline to the master control valve 4060 and the master control valve is positioned to seal off (close) its other ports, including the output port communicating with vacuum lumen 108. By closing the port to the lateral lumen 108 whole in the Ready State, adow through the device may be minimized, which may allow the pump 4010 to more easily maintain the desired vacuum level at the vacuum canister 4030.

When the operator depresses the "Sample" button 170 in the present example, the cutter motor is activated to cause the cutter 120 to retract proximally. As the cutter retracts, the valve 4080 changes position to communicate vacuum to the master control valve 4060. At the same time, the master control valve changes position to communicate a vacuum to the vacuum lumen 108. With the tissue aperture 1 14 open, vacuum from vacuum pump 401 0 is applied to the cutter 120 (such as via the tissue storage assembly 2000) and cutter lumen 104 (via the cutter 120), as well as to the vacuum lumen 108 (via the valves 4080 and 4060). Vacuum applied to both cutter lumen 104 and vacuum lumen 108 assists in prolapsing tissue into aperture 114 of cannula 100.

After maintaining this vacuum state for a second or more to ensure tissue has prolapsed into aperture 114, the cutter 120 is advanced distally (and simultaneously rotated) to close the aperture 114 and severe a tissue sample in the distal portion of the hollow cutter 120. As the cutter 120 advances distally, the cutter 120 can contact or otherwise actuate the valve 4080 to change the valve position to communicate saline to the master control valve 4060. Also, as the cutter 120 advances, a microprocessor can be employed to change the master control valve 4060 position to communicate filtered atmospheric air to vacuum lumen 108, which in turn is communicated via passageways 107, 109 to the distal face of the severed tissue sample positioned in the distal portion of hollow cutter 120. The atmospheric air on the distal face of the tissue sample provides a proximal pushing force on the tissue sample, while the vacuum provided in cutter 120 (via the tissue storage assembly 2000) provides a proximally directed pulling force on the severed tissue sample. The resulting proximally directed force on the tissue sample conveys the tissue sample through the hollow cutter 120 into tissue storage assembly 2000. Of course, any other suitable structures or techniques may be used to capture a tissue sample and communicate it to a tissue storage assembly 2000.

In an alternative embodiment, the microprocessor can be employed to change the position of master control valve 4060 to first communicate saline to vacuum lumen 108 for a predetermined period of time, and then change the valve's position to communicate atmospheric air to the lumen 108. Accordingly, a fixed volume of saline can be delivered via passageways 107,109 to the distal end of hollow cutter 120, thereby assisting in moving the severed tissue sample proximally through hollow cutter 120 to tissue storage assembly 2000. The control system can be programmed to return to the Ready State after a predetermined period of time (e.g., one or more seconds).

The biopsy device operator can depress the "Clear Probe" button 172 while in the Ready State (e.g., after having operated the "Sample" button 170 to sever tissue) in order to direct a microprocessor control to cause the cutter 120 to reciprocate slightly to open and close aperture 1 14 a fraction of an inch (e.g. 5 mm (0.2 inches)), or to any suitable degree. This reciprocation of cutter 120 can be effective to dislodge the tissue sample or otherwise free the sample so that the sample can travel freely through hollow cutter 120. While the cutter 120 is reciprocating, the vacuum control valve 4060 can be repositioned to communicate saline to the vacuum lumen 108 and through passageways 107, 109 to provide a pulsing force on the distal face of the tissue sample. After a predetermined period of time, the microprocessor can return the pneumatic system to the Ready State.

The operator can depress and release the "Aspirate/Insert" button 174 when the device is in the Ready State to insert medication or a tissue marker into the tissue being sampled or into the site from which a tissue sample has been or will be taken. When the button 174 is depressed in this example, the cutter 120 moves proximally to open aperture 114. The position of the master control valve 4060 is changed to communicate atmospheric air to the vacuum lumen 108. Depressing the "Aspirate/Insert" button 174 also turns off the vacuum (such as by either turning off the pump 4010 or opening regulator 4020 to vent pump 4010 outlet to atmosphere, etc.). The tissue marker applier (or medication) can be fed into the proximal end of the cannula 100 through hollow cutter 120, such as via the tissue storage assembly 2000 or otherwise, with the marker (or medication) being then deployed through the open aperture 114 in cannula 100. After the marker or medication has been deployed, the user may press any button, which may advance the cutter 120 to return to the Ready State with the master control valve 4060 positioned up.

The operator can depress and hold the "Aspirate/Insert" button 174 to aspirate fluid in the vicinity of aperture 1 14. When the operator depresses the button 174 in this example, the cutter 120 moves proximally to open aperture 1 14. With the cutter positioned proximally, the valve 4080 communicates vacuum to the master control valve 4060, and the master control valve 4060 is positioned to communicate vacuum to the vacuum lumen 108. Accordingly, vacuum is applied to both the lumen 108 and the cutter lumen 104 (because vacuum is provided continuously through cutter 120 to lumen 104 while the pump 4010 operates in this example). The vacuum provided to lumen 104 and lumen 108 aspirates any liquid near the aperture 1 14. When the "Aspirate/Insert" button 174 is released, the pneumatic system is controlled to return the Ready State. The cutter 120 is advanced to close aperture 114. As the cutter 120 is advanced in this example, the master control valve 4060 is positioned to communicate filtered atmospheric air to the vacuum lumen 108. Once the aperture 114 is closed, the master control valve 4060 is positioned to close all its ports to attain the Ready State. As with other operational sequences described herein, the foregoing operational sequence is merely illustrative, and any other suitable operational sequences may be used in addition to or in lieu of those explicitly described herein.

The length of the umbilicus 200 from the control module 5000 housing the vacuum pump 40 10 to the biopsy device 10 can be relatively long (as much as 6.1 m (20 feet) or more in some cases) in order to accommodate movement of the biopsy device 10 in the operating room, or due to limitations of the position of the control module 5000 in magnetic resonance imaging environments. Of course, umbilicus 200 may be of any desired length. In the present example, the vacuum line in the umbilicus 200 can account for a considerable portion of the flow volume that needs to be supplied or maintained by the vacuum pump 4010 and vacuum canister 4030 when the tissue aperture 114 is open. Placing the saline vacuum valve 4080 and the master control valve 4080 at the distal end of the vacuum line (the end associated with the biopsy device 10) instead of in the biopsy vacuum control unit 5000 may mean that a smaller vacuum pump 401 0 and a smaller vacuum canister 4030 can be used. In some conventional biopsy devices, valving may be placed in the control unit that includes the vacuum pump, and the control unit is may be mounted on wheels due to its weight and size. In Figures 2-4, the valves 4060 and 4080 are shown disposed in the biopsy device 10. The valves can be disposed in a disposable probe portion 160, 163 that includes the cannula 100 and cutter 120 and/or in a non-disposable (e.g., in the holster 130) portion of the biopsy device 10. Such a valve placement may allow a relatively low weight diaphragm vacuum pump 4010 having a flow rate of about 18 liters per minute to be used, as compared to a conventional pump and valve arrangement requiring more than 80 liters per minute. Of course, any desired vacuum pump having any desired properties may be used.

Similarly, the vacuum canister 4030 can be relatively small, with a volume of less than about 300 cubic centimeters, as compared to a conventional vacuum canister having a volume storage capacity of 1200 cc's or more. As a result, a relatively lightweight, hand-portable vacuum control module 5000 can be employed. The vacuum control module 5000 (Figures 1,2, and 5) can weigh less than 11 kg (25 pounds), can be carried by one hand, and can have height, width, and length dimensions each less than about 55 cm (1.5 feet). Alternatively, vacuum canister 4030 and control module 5000 may have any other suitable capacity, size, weight, or other properties.

If desired, a foot pedal (not shown) or remote keypad (not shown) can be employed to provide control input or instructions to the biopsy device 10 directly and/or to the vacuum control module 5000, such as via a connector 180. The foot pedal and remote keypad can be tethered (e.g., with one or more wires extending from the food pedal/ keypad to the vacuum control module 5000, etc.). Alternatively, "wireless" communication between the foot pedal keypad and the control module 5000 and/or the biopsy device 10 can be employed. For instance, wireless "Bluetooth" communication and associated hardware and software can be employed to provide wireless control signals to the vacuum control module 5000 and/or the biopsy device 10 without requiring a "line of sight" for signal transmission and reception. Alternatively, an Infrared transmitter and receiver can be employed to send and receive control signals. Other ways in which communication may be provided between components of a biopsy system (e.g., between a pedal keypad and control module 5000), whether wired, wireless, or otherwise, will be apparent to those of ordinary skill in the art in view of the teachings herein.

While preferred embodiments of the present invention have been shown and described herein, it will be obvious to those skilled in the art that such embodiments are provided by way of example only. Numerous variations, changes, and substitutions will now occur to those skilled in the art without departing from the scope of the appended claims.

## Claims

1. A vacuum system adapted to be used with a biopsy device (10), the vacuum system comprising:
(a) a vacuum control module (5000), wherein the vacuum control module comprises:
i) a vacuum pump (4010), and **characterised by**:
ii) a canister receiving portion (5030), wherein the canister receiving portion has a first port (5033) in fluid communication with the vacuum pump (4010);
(b) a canister (4030), wherein the canister is configured to be engaged with the canister receiving portion of the vacuum control module (5000), wherein the canister comprises:
i) a reservoir (4032), wherein the reservoir is configured to hold fluid, and
ii) a second port (4033), wherein the second port is in fluid communication with the reservoir, wherein the second port is configured to engage directly with the first port (5033) of the vacuum control module to provide fluid communication between the reservoir and the vacuum pump upon engagement of the canister with the canister receiving portion of the vacuum control module;
(d) a conduit (200) adapted to be coupled with a biopsy device (10) and the canister (4030), wherein the conduit defines a conduit lumen configured to provide fluid communication between the reservoir of the canister and at least one lumen of the biopsy device (10).

2. A vacuum system of claim 1, wherein the conduit (200) comprises:
a first conduit (202) configured to communicate vacuum from the reservoir to the biopsy device; and
a second conduit (206) configured to communicate saline to the biopsy device, wherein the second conduit is substantially parallel with the first conduit.

3. A biopsy system, comprising:
a biopsy device (10), wherein the biopsy device (10) comprises:
i) a cannula (100),
ii) a cutter (120), wherein the cutter is configured to sever tissue, and
iii) one or more lumens (104, 108) configured to communicate fluid;
and
the vacuum system of claim 1.

4. The biopsy system of Claim 3, wherein the cutter (120) comprises a hollow tube, wherein the hollow tube defines a first lumen of the one or more lumens.

5. The biopsy system of Claim 4, wherein the cannula (100) defines a second lumen of the one or more lumens, wherein the second lumen is parallel with the first lumen.

6. The biopsy system of Claim 3, wherein the first port (5033) has a female configuration, wherein the second port (4033) has a male configuration.

7. The biopsy system of Claim 3, wherein the canister receiving portion (5030) comprises an opening defined by the vacuum control module, wherein the opening is sized for insertion of the canister therethrough.

8. The biopsy system of Claim 3, wherein the canister (4030) further comprises a float (4063), wherein the float is configured to close the second port in response to fluid reaching a certain level within the reservoir.

9. The biopsy system of Claim 3, wherein the vacuum control module (5000) further comprises a power source, wherein the conduit (200) further comprises a cable configured to communicate power from the power source to the biopsy device.

10. The biopsy system of Claim 9, wherein the cable and the conduit lumen are integrally formed in the conduit (200).

11. The biopsy system of Claim 9, wherein the vacuum control module (5000) further comprises a first set of contacts (5023) in communication with the power source, wherein the canister further comprises a second set of contacts (223) in communication with the cable, wherein the first set of contacts and the second set of contacts are configured to engage upon engagement of the canister (4030) with the canister receiving portion (5030) of the vacuum control module (5000) to provide electrical communication between the power source and the biopsy device.

## Patentansprüche

1. Vakuumsystem zur Verwendung mit einem Biopsiegerät (10), wobei das Vakuumsystem umfasst:
(a) ein Vakuumkontrollmodul (5000), wobei das Vakuumkontrollmodul umfasst:
i) eine Vakuumpumpe (4010), **gekennzeichnet durch**
ii) einen Kanisteraufnahmeabschnitt (5030), wobei der Kanisteraufnahmeabschnitt einen ersten Anschluss (5033) in Flüssigkeitsverbindung mit der Vakuumpumpe (4010) aufweist;
(b) einen Kanister (4030), wobei der Kanister für einen Eingriff mit dem Kanisteraufnahmeabschnitt des Vakuumkontrollmoduls (5000) konfiguriert ist, wobei der Kanister umfasst:
i) einen Behälter (4032), wobei der Behälter zur Aufnahme von Flüssigkeit konfiguriert ist, und
ii) einen zweiten Anschluss (4033), wobei der zweite Anschluss in Flüssigkeitsverbindung mit dem Behälter steht, wobei der zweite Anschluss zum direkten Eingriff mit dem ersten Anschluss (5033) des Vakuumkontrollmoduls zum Bereitstellen einer Flüssigkeitsverbindung zwischen dem Behälter und der Vakuumpumpe bei Eingriff des Kanisters mit dem Kanisteraufnahmeabschnitt des Vakuumkontrollmoduls konfiguriert ist;
(d) eine Leitung (200), die gestaltet ist, um mit einem Biopsiegerät (10) und dem Kanister (4030) gekoppelt zu werden, wobei die Leitung ein Leitungslumen definiert, das zum Bereitstellen einer Flüssigkeitsverbindung zwischen dem Behälter des Kanisters und mindestens einem Lumen des Biopsiegeräts (10) konfiguriert ist.

2. Vakuumsystem nach Anspruch 1, **dadurch gekennzeichnet, dass** die Leitung (200) umfasst:
eine erste Leitung (202), die zum Übertragen von Vakuum vom Behälter zum Biopsiegerät konfiguriert ist; und
eine zweite Leitung (206), die zum Übertragen von physiologischer Kochsalzlösung zum Biopsiegerät konfiguriert ist, wobei die zweite Leitung im wesentlichen parallel zur ersten Leitung verläuft.

3. Biopsiesystem, umfassend:
ein Biopsiegerät (10), wobei das Biopsiegerät (10) umfasst:
i) eine Kanüle (100),
ii) eine Schneideinrichtung (120), wobei die Schneideinrichtung zum Abtrennen von Gewebe konfiguriert ist, und
iii) ein oder mehrere Lumen (104, 108), das/die zum Übertragen von Flüssigkeit konfiguriert ist/sind;
und
das Vakuumsystem nach Anspruch 1.

4. Biopsiesystem nach Anspruch 3, **dadurch gekennzeichnet, dass** die Schneideinrichtung (120) ein hohles Rohr umfasst, wobei das hohle Rohr ein erstes Lumen des einen oder der mehreren Lumen definiert.

5. Biopsiesystem nach Anspruch 4, **dadurch gekennzeichnet, dass** die Kanüle (100) ein zweites Lumen des einen oder der mehreren Lumen definiert, wobei das zweite Lumen parallel zum ersten Lumen verläuft.

6. Biopsiesystem nach Anspruch 3, **dadurch gekennzeichnet, dass** der erste Anschluss (5033) eine weibliche Konfiguration aufweist, wobei der zweite Anschluss (4033) eine männliche Konfiguration aufweist.

7. Biopsiesystem nach Anspruch 3, **dadurch gekennzeichnet, dass** der Kanisteraufnahmeabschnitt (5030) eine Öffnung umfasst, die von dem Vakuumkontrollmodul definiert ist, wobei die Öffnung zum Einsetzen des Kanisters durch selbige dimensioniert ist.

8. Biopsiesystem nach Anspruch 3, **dadurch gekennzeichnet, dass** der Kanister (4030) ferner einen Schwimmer (4063) umfasst, wobei der Schwimmer zum Verschließen des zweiten Anschlusses als Reaktion darauf, dass Flüssigkeit einen bestimmten Pegel in dem Behälter erreicht, konfiguriert ist.

9. Biopsiesystem nach Anspruch 3, **dadurch gekennzeichnet, dass** das Vakuumkontrollmodul (5000) ferner eine Stromquelle umfasst, wobei die Leitung (200) ferner ein Kabel umfasst, das zum Übertragen von Energie von der Stromquelle zum Biopsiegerät konfiguriert ist.

10. Biopsiesystem nach Anspruch 9, **dadurch gekennzeichnet, dass** das Kabel und das Leitungslumen in der Leitung (200) einteilig ausgebildet sind.

11. Biopsiesystem nach Anspruch 9, **dadurch gekennzeichnet, dass** das Vakuumkontrollmodul (5000) ferner einen ersten Satz von Kontakten (5023) in Verbindung mit der Stromquelle umfasst, wobei der Kanister ferner einen zweiten Satz von Kontakten (223) in Verbindung mit dem Kabel umfasst, wobei der erste Satz von Kontakten und der zweite Satz von Kontakten für einen Eingriff bei Eingriff des Kanisters (4030) mit dem Kanisteraufnahmeabschnitt (5030) des Vakuumkontrollmoduls (5000) konfiguriert sind, um eine elektrische Verbindung zwischen der Stromquelle und dem Biopsiegerät bereitzustellen.

## Revendications

1. Système de vide adapté pour être utilisé avec un dispositif de biopsie (10), le système de vide comprenant :
(a) un module de commande de vide (5000), dans lequel le module de commande de vide comprend :
i) une pompe à vide (4010), et **caractérisé par** :
ii) une partie de réception d'absorbeur (5030), dans lequel la partie de réception d'absorbeur a un premier orifice (5033) en communication de fluide avec la pompe à vide (4010) ;
(b) un absorbeur (4030), dans lequel l'absorbeur est configuré pour être mis en prise avec la partie de réception d'absorbeur du module de commande de vide (5000), dans lequel l'absorbeur comprend :
i) un réservoir (4032), dans lequel le réservoir est configuré pour contenir.du fluide, et
ii) un second orifice (4033), dans lequel le second orifice est en communication de fluide avec le réservoir, dans lequel le second orifice est configuré pour se mettre en prise directement avec le premier orifice (5033) du module de commande de vide afin de fournir la communication de fluide entre le réservoir et la pompe à vide suite à la mise en prise de l'absorbeur avec la partie de réception d'absorbeur du module de commande de vide ;
(d) un conduit (200) adapté pour être couplé avec un dispositif de biopsie (10) et l'absorbeur (4030), dans lequel le conduit définit une lumière de conduit configurée pour fournir la communication de fluide entre le réservoir de l'absorbeur et au moins une lumière du dispositif de biopsie (10).

2. Système de vide selon la revendication 1, dans lequel le conduit (200) comprend :
un premier conduit (202) configuré pour communiquer le vide du réservoir jusqu'au dispositif de biopsie ; et
un second conduit (206) configuré pour communiquer une solution saline au dispositif de biopsie, dans lequel le second conduit est sensiblement parallèle au premier conduit.

3. Système de biopsie, comprenant :
un dispositif de biopsie (10), dans lequel le dispositif de biopsie (10) comprend :
i) une canule (100),
ii) un dispositif de coupe (120), dans lequel le dispositif de coupe est configuré pour sectionner le tissu, et
iii) une ou plusieurs lumières (104, 108) configurées pour communiquer du fluide ; et
le système de vide selon la revendication 1.

4. Système de biopsie selon la revendication 3, dans lequel le dispositif de coupe (120) comprend un tube creux, dans lequel le tube creux définit une première lumière des une ou plusieurs lumières.

5. Système de biopsie selon la revendication 4, dans lequel la canule (100) définit une seconde lumière des une ou plusieurs lumières, dans lequel la seconde lumière est parallèle à la première lumière.

6. Système de biopsie selon la revendication 3, dans lequel le premier orifice (5033) a une configuration femelle, dans lequel le second orifice (4033) a une configuration mâle.

7. Système de biopsie selon la revendication 3, dans lequel la partie de réception d'absorbeur (5030) comprend une ouverture définie par le module de commande de vide, dans lequel l'ouverture est dimensionnée pour l'insertion de l'absorbeur à travers cette dernière.

8. Système de biopsie selon la revendication 3, dans lequel l'absorbeur (4030) comprend en outre un flotteur (4063), dans lequel le flotteur est configuré pour fermer le second orifice en réponse au fluide qui atteint un certain niveau à l'intérieur du réservoir.

9. Système de biopsie selon la revendication 3, dans lequel le module de commande de vide (5000) comprend en outre une source de puissance, dans lequel le conduit (200) comprend en outre un câble configuré pour communiquer la puissance de la source de puissance au dispositif de biopsie.

10. Système de biopsie selon la revendication 9, dans lequel le câble et la lumière de conduit sont formés de manière solidaire dans le conduit (200).

11. Système de biopsie selon la revendication 9, dans lequel le module de commande de vide (5000) comprend en outre un premier ensemble de contacts (5023) en communication avec la source de puissance, dans lequel l'absorbeur comprend en outre un second ensemble de contacts (223) en communication avec le câble, dans lequel le premier ensemble de contacts et le second ensemble de contacts sont configurés pour se mettre en prise suite à la mise en prise de l'absorbeur (4030) avec la partie de réception d'absorbeur (5030) du module de commande de vide (5000) pour fournir la communication électrique entre la source de puissance et le dispositif de biopsie.
